(19) Europäisches Patentamt
European Patent Office
Office européen des brevets

(11) Veröffentlichungsnummer: **0 077 301**
**B1**

(12) # EUROPÄISCHE PATENTSCHRIFT

(45) Veröffentlichungstag der Patentschrift: **21.08.85**

(51) Int. Cl.⁴: **C 07 F 7/22**, A 01 N 55/04

(21) Anmeldenummer: **82810417.4**

(22) Anmeldetag: **07.10.82**

(54) **Triorganozinn-Isocyanurverbindungen, ein Verfahren zu ihrer Herstellung und ihre Verwendung in der Schädlingsbekämpfung.**

(30) Priorität: **13.10.81 CH 6544/81**

(43) Veröffentlichungstag der Anmeldung: **20.04.83 Patentblatt 83/16**

(45) Bekanntmachung des Hinweises auf die Patenterteilung: **21.08.85 Patentblatt 85/34**

(84) Benannte Vertragsstaaten: **AT BE CH DE FR GB IT LI NL**

(56) Entgegenhaltungen:
**GB - A - 1 021 200**

**THE JOURNAL OF ORGANIC CHEMISTRY, Band 30, Nr. 3, 12. März 1965, Seiten 693-695 W. STAMM: "Organometallic isocyanates and isocyanates and isocyanurates of groups IV and V"**
**JOURNAL OF THE CHEMICAL SOCIETY, C. 1966, Seiten 1311-1315 A.G. DAVIES et al.: "Organometallic reactions. part V. Trialkylstannylation of amides"**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Wehner, Wolfgang, Dr., Wetzbach 34, D-6144 Zwingenberg (DE)**
Erfinder: **Ackermann, Peter, Dr., Hangelimattweg 113, CH-4148 Pfeffingen (CH)**

**EP 0 077 301 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Beschreibung

Die vorliegende Erfindung betrifft Triorganozinn-isocyanurverbindungen, Verfahren zu ihrer Herstellung und ihre Verwendung in der Schädlingsbekämpfung.

Die Triorganozinn-isocyanurverbindungen haben die Formel

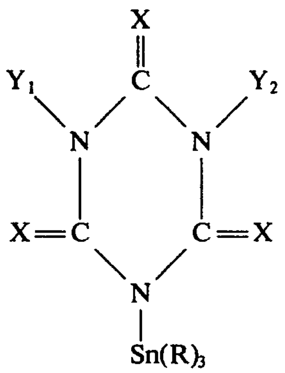

(I)

worin
R Cyclohexyl oder

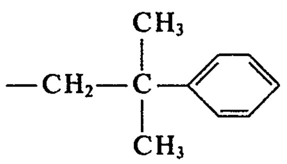

X Sauerstoff oder Schwefel und
$Y_1$ und $Y_2$ je Wasserstoff oder $-Sn(R)_3$ bedeuten.

Bevorzugt sind Verbindungen der Formel I, worin
R Cyclohexyl oder

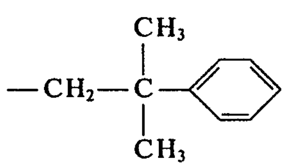

X Sauerstoff oder Schwefel und
$Y_1$ und $Y_2$ je $-Sn(R)_3$ bedeuten.

Die Verbindungen der Formel I können nach an sich bekannten Methoden z. B. wie folgt hergestellt werden

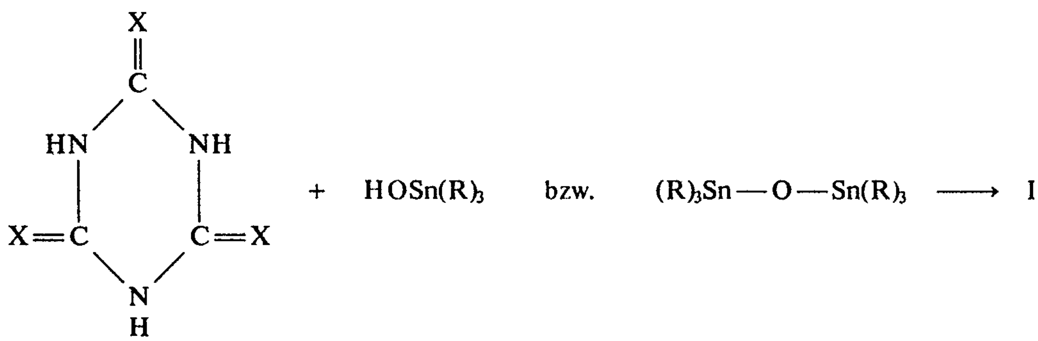


(II) (III) (IV)

In den Formeln II, III und IV haben R und X die für die Formel I angegebene Bedeutung.

Das Verfahren wird zweckmäßig bei einer Temperatur zwischen 30 bis 180°C, vorzugsweise bei 80 bis 150°C, bei normalem oder leicht reduziertem Druck und vorzugsweise in Gegenwart eines gegenüber den Reaktionsteilnehmern inerten Lösungs- oder Verdünnungsmittels durchgeführt.

Als Lösungs- oder Verdünnungsmittel eignen sich z. B. nicht halogenierte oder halogenierte Kohlenwasserstoffe, wie Petroläther, Toluol, Chloroform oder Methylenchlorid; Alkohole wie Methanol oder

Äthanol oder Isopropanol; Äther und ätherartige Verbindungen wie Diäthyläther, Dioxan und Tetrahydrofuran; Ketone wie Aceton, Cyclohexanon oder Methyläthylketon. Die Ausgangsstoffe der Formeln II bis IV sind bekannt und können nach bekannten Methoden hergestellt werden.

Die Verbindungen der Formel I eignen sich zur Bekämpfung von verschiedenartigen Schädlingen an Tieren und Pflanzen. Sie haben auch pflanzenwuchsregulierende Wirkung.

Insbesondere eignen sich die Verbindungen der Formel I zur Bekämpfung aller Entwicklungsstadien von phytopathogenen und tierparasitären Insekten, z. B. der Ordnungen Lepidoptera, Coleoptera, Homoptera, Diptera, Thysanoptera, Orthoptera, Anoplura, Siphonaptera, Mallophaga, Thysanura, Isoptera, Psocoptera und Hymenoptera und von tierparasitären und phytopathogenen Milben und Zecken der Ordnung Acarina.

Vor allem eignen sich Verbindungen der Formel I besser zur Bekämpfung von pflanzenschädigenden Milben (Tetranychus cinnabarinus) als die in der britischen Patentschrift Nr. 1 021 200 und im »Journal of Organic Chemistry 30 (1965) 694« beschriebenen 1,3,5-Trialkyl-, -phenyl- oder -benzylzinntriazin-trione.

Die Verbindungen der Formel I werden in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnußöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}-C_{22}$), wie z. B. die Na- oder K-Salze der Öl- oder Stearinsäure, oder von natürlichen Fettsäuregemischen, die z. B. aus Kokosnuß- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit 8 bis 22 C-Atomen; Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage,

die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxid-Addukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)äthylammoniumbromid.

Die in der Formulierungstechnik gebräuchlichen Tenside sind u. a. in folgender Publikation beschrieben: »McCutcheon's Detergents and Emulsifiers Annual« MC Publishing Corp., Ridgewood New Jersey, 1979.

Die pestiziden Zubereitungen enthalten in der Regel 0,1 bis 99%, insbesondere 0,1 bis 95%, Wirkstoff der Formel I, 1 bis 99,9% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I
(% = Gewichtsprozent)

| 1. Emulsions-Konzentrate | a) | b) | c) |
|---|---|---|---|
| Wirkstoff | 20% | 40% | 50% |
| Ca-Dodecylbenzolsulfonat | 5% | 8% | 5,8% |
| Ricinusöl-polyäthylenglykoläther (36 Mol AeO) | 5% | – | – |
| Tributylphenol-polyäthylenglykoläther (30 Mol AeO) | – | 12% | 4,2% |
| Cyclohexanon | – | 15% | 20% |
| Xylolgemisch | 70% | 25% | 20% |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff | 80% | 10% | 5% | 95% |
| Äthylenglykol-monomethyläther | 20% | – | – | – |
| Polyäthylenglykol MG 400 | – | 70% | – | – |
| N-Methyl-2-pyrrolidon | – | 20% | – | – |
| Epoxidiertes Kokosnußöl | – | – | 1% | 5% |
| Benzin (Siedegrenzen 160–190°C) | – | – | 94% | – |

Die Lösungen sind zur Anwendung in Form kleinster Tropfen geeignet.

| 3. Granulate | a) | b) |
|---|---|---|
| Wirkstoff | 5% | 10% |
| Kaolin | 94% | – |
| Hochdisperse Kieselsäure | 1% | |
| Attapulgit | – | 90% |

Der Wirkstoff wird in Methylenchlorid gelöst, auf den Träger aufgesprüht und das Lösungsmittel anschließend im Vakuum abgedampft.

4. Stäubemittel

| | a) | b) |
|---|---|---|
| Wirkstoff | 2% | 5% |
| Hochdisperse Kieselsäure | 1% | 5% |
| Talkum | 97% | – |
| Kaolin | – | 90% |

Durch inniges Vermischen der Trägerstoffe mit dem Wirkstoff erhält man gebrauchsfertige Stäubemittel.

Formulierungsbeispiele für feste Wirkstoffe der Formel I
(% = Gewichtsprozent)

5. Spritzpulver

| | a) | b) |
|---|---|---|
| Wirkstoff | 20% | 60% |
| Na-Ligninsulfonat | 5% | 5% |
| Na-Laurylsulfat | 3% | – |
| Na-Diisobutylnaphthalinsulfonat | – | 6% |
| Octylphenolpolyäthylenglykoläther (7–8 Mol AeO) | – | 2% |
| Hochdisperse Kieselsäure | 5% | 27% |
| Kaolin | 67% | – |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrat

| | |
|---|---|
| Wirkstoff | 10% |
| Octylphenolpolyäthylenglykoläther (4–5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

7. Stäubemittel

| | a) | b) |
|---|---|---|
| Wirkstoff | 5% | 8% |
| Talkum | 95% | – |
| Kaolin | – | 92% |

Man erhält anwendungsfertige Stäubemittel, indem der Wirkstoff mit dem Träger vermischt und auf einer geeigneten Mühle vermahlen wird.

8. Extruder Granulat

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschließend im Luftstrom getrocknet.

9. Umhüllungs-Granulat

| | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmäßig aufgetragen. Auf diese Weise erhält man staubfreie Umhüllungs-Granulate.

10. Suspensions-Konzentrat

| | |
|---|---|
| Wirkstoff | 40% |
| Äthylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wäßrige Formaldehydlösung | 0,2% |
| Silikonöl in Form einer 75%igen wäßrigen Emulsion | 0,8% |
| Wasser | 32% |

Beispiel 1

Herstellung von Tris-tricyclohexylzinn-isocyanurat

Eine Suspension aus 193,7 g Cyanursäure und 173,3 g Tricyclohexylzinnhydroxid werden in 6 Liter Toluol 6 Stunden am Rückfluß gekocht, wobei das entstehende Reaktionswasser azeotropisch abdestilliert wird. Nach beendeter Reaktion wird das Produkt abfiltriert und getrocknet. Man erhält die Verbindung der Formel

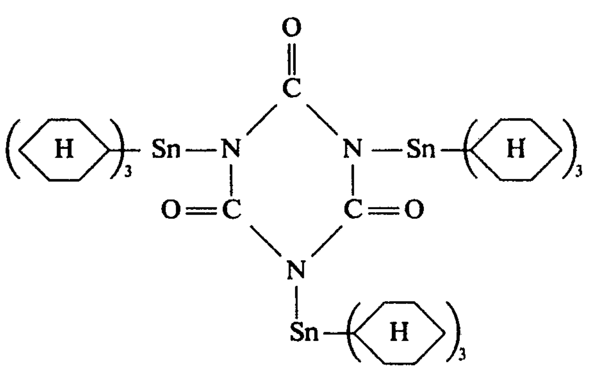

mit einem Schmelzpunkt von >350°C.

Auf analoge Weise werden auch folgende Verbindungen hergestellt:

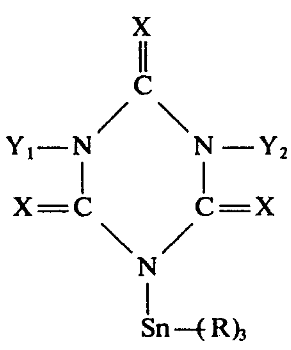

| R | X | $Y_1$ | $Y_2$ | Physikalische Daten |
|---|---|---|---|---|
| 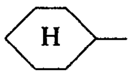 | S | —Sn—(R)$_3$ | —Sn—(R)$_3$ | Smp. 188–189°C |
| 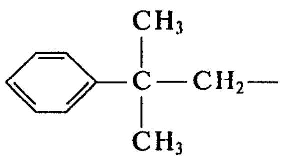 | S | —Sn—(R)$_3$ | —Sn—(R)$_3$ | NMR-Spektrum $\delta$ $119_{Sn}$ = 2,60 ppm |
| 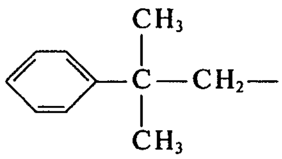 | O | —Sn—(R)$_3$ | —Sn—(R)$_3$ | Smp. 131–132°C |
| 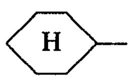 | O | —Sn—(R)$_3$ | H | |
| 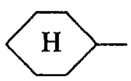 | O | H | H | |

## Beispiel 2

### Wirkung gegen Anthonomus grandis

Baumwollpflanzen im 4 bis 5-Blattstadium werden mit einer Versuchslösung enthaltend 25 und 50 g des zu prüfenden Wirkstoffes pro 100 l Wasser bis zur Tropfnässe besprüht.

Nach dem Antrocknen des Spritzbelages werden die Pflanzen je mit 5 Anthonomus grandis-Käfern besetzt. Man verwendet pro Versuchsverbindung und pro Konzentration zwei Pflanzen. Eine Auswertung erfolgt nach 24, 48 und 120 Stunden. Der Versuch wird bei 24°C und bei 60% relativer Luftfeuchtigkeit durchgeführt. Verbindungen gemäß dem Herstellungsbeispiel zeigen eine gute Wirkung gegenüber Anthonomus grandis.

## Beispiel 3

### Wirkung gegen pflanzenschädigende Akariden: Tetranychus urticae (OP-sensible) und Tetranychus cinnabarinus (OP-tolerant)

Die Primärblätter von Phaseolus vulgaris Pflanzen werden 16 Stunden vor dem Versuch auf akarizide Wirkung mit einem infestierten Blattstück aus einer Massenzucht von Tetranychus urticae (OP-sens.) oder Tetranychus cinnabarinus (OP-tol.) belegt (die Toleranz bezieht sich auf die Verträglichkeit von Diazinon).

Die so behandelten infestierten Pflanzen werden mit einer Versuchslösung enthaltend 400 oder 200 ppm der zu prüfenden Verbindung bis zur Tropfnässe besprüht.

Nach 24 Stunden und wiederum nach 7 Tagen werden Imagines und Larven (alle beweglichen Stadien) unter dem Binokular auf lebende und tote Individuen ausgewertet.

Man verwendete pro Konzentration und pro Testspezies eine Pflanze. Während des Versuchsverlaufs stehen die Pflanzen in Gewächshauskabinen bei 25°C.

Bei der Konzentration von 200 ppm zeigen die Verbindungen gemäß dem Herstellungsbeispiel 100%ige Wirkung gegen Individuen der Spezies Tetranychus urticae und Tetranychus cinnabarinus.

**Patentansprüche für die Vertragsstaaten BE, CH, DE, FR, GB, IT, LI, NL**

1. Triorganozinn-isocyanurverbindungen der Formel

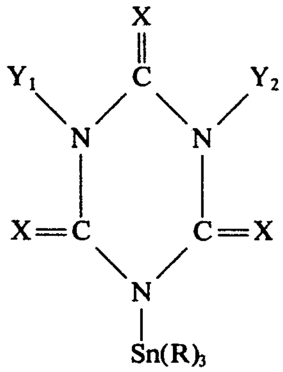

(I)

worin
R Cyclohexyl oder

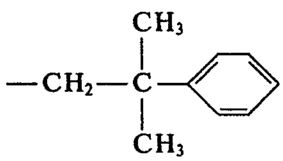

X Sauerstoff oder Schwefel und
$Y_1$ und $Y_2$ je Wasserstoff oder $-Sn(R)_3$ bedeuten.

2. Eine Verbindung gemäß Anspruch 1, worin
R Cyclohexyl oder

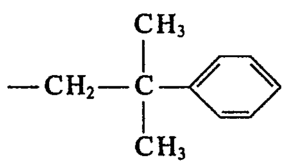

X Sauerstoff oder Schwefel und
$Y_1$ und $Y_2$ je $-Sn(R)_3$ bedeuten.

3. Die Verbindung gemäß Anspruch 2 der Formel

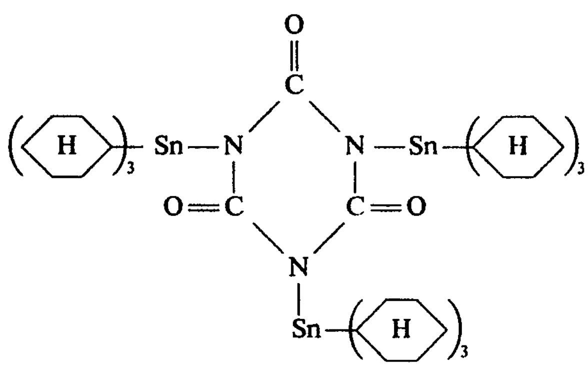

4. Die Verbindung gemäß Anspruch 2 der Formel

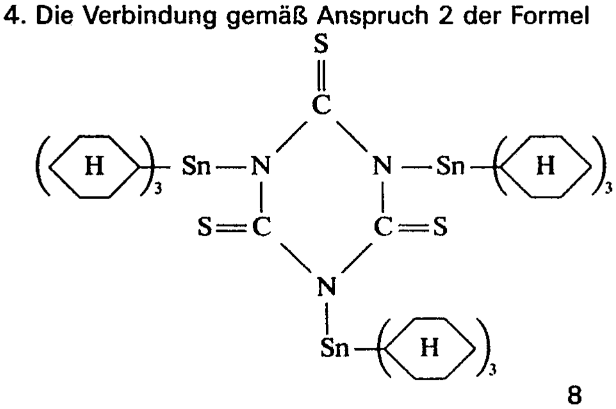

5. Die Verbindung gemäß Anspruch 2 der Formel

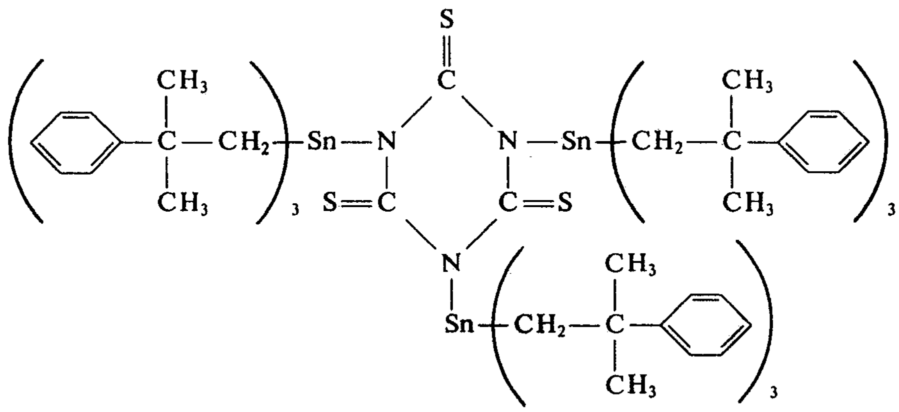

6. Ein Verfahren zur Herstellung von Verbindungen gemäß Anspruch 1, dadurch gekennzeichnet, daß man eine Verbindung der Formel

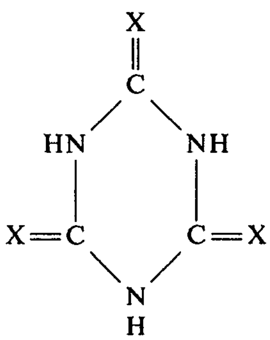

mit einer Verbindung der Formel

$HOSn(R)_3$ bzw. $(R)_3Sn - O - Sn(R)_3$

umsetzt, worin R und X die im Anspruch 1 angegebene Bedeutung haben.

7. Ein Schädlingsbekämpfungsmittel, welches als aktive Komponente eine Verbindung gemäß Anspruch 1 enthält.

8. Die Verwendung einer Verbindung gemäß Anspruch 1 zur Bekämpfung von verschiedenartigen Schädlingen an Tieren und Pflanzen sowie zur Regulierung des Pflanzenwachstums.

9. Die Verwendung gemäß Anspruch 8 zur Bekämpfung von Insekten und Vertretern der Ordnung Akarina.

**Patentansprüche für den Vertragsstaat AT**

1. Ein Schädlingsbekämpfungsmittel, welches als aktive Komponente eine Triorganozinn-isocyanurverbindung der Formel

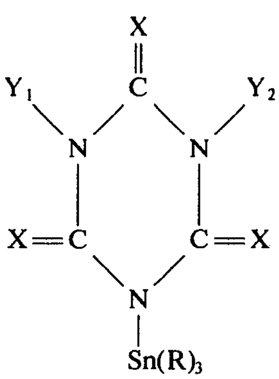

(I)

enthält, worin
R Cyclohexyl oder

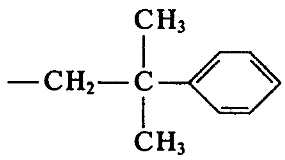

X Sauerstoff oder Schwefel und
$Y_1$ und $Y_2$ je Wasserstoff oder – $Sn(R)_3$ bedeuten.

2. Ein Mittel gemäß Anspruch 1, worin in der Formel der aktiven Verbindung
R Cyclohexyl oder

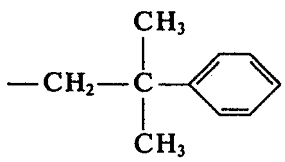

X Sauerstoff oder Schwefel und
$Y_1$ und $Y_2$ je $-Sn(R)_3$ bedeuten.

3. Ein Mittel gemäß Anspruch 2, welches als aktive Komponente die Verbindung der Formel

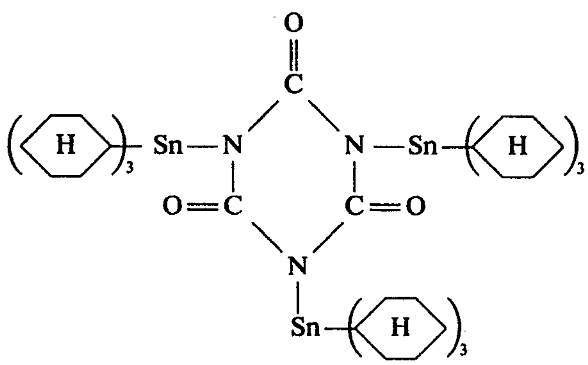

enthält.

4. Ein Mittel gemäß Anspruch 2, welches als aktive Komponente die Verbindung der Formel

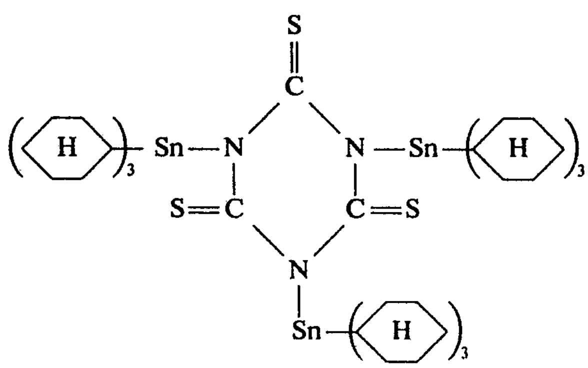

enthält.

5. Ein Mittel gemäß Anspruch 2, welches als aktive Komponente die Verbindung der Formel

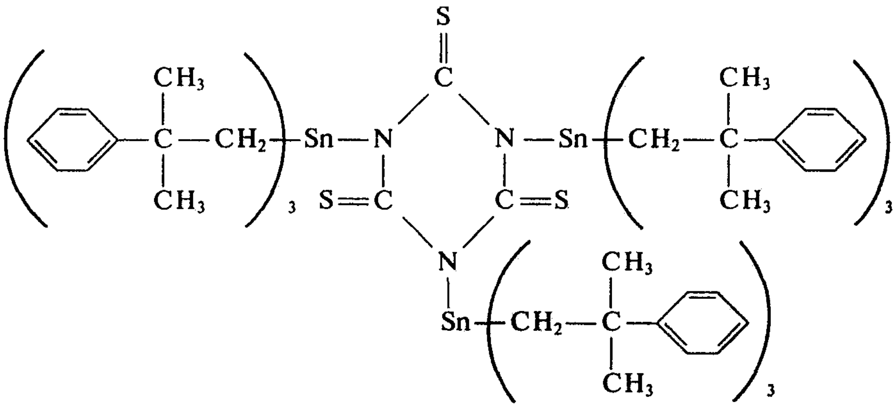

enthält.

6. Die Verwendung des Mittels gemäß Anspruch 1 zur Bekämpfung von verschiedenartigen Schädlingen an Tieren und Pflanzen sowie zur Regulierung des Pflanzenwachstums.

7. Die Verwendung gemäß Anspruch 6 zur Bekämpfung von Insekten und Vertretern der Ordnung Akarina.

8. Verfahren zur Herstellung von Triorganozinn-isocyanurverbindungen der Formel

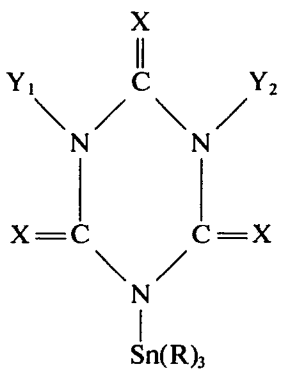

(I)

worin R Cyclohexyl oder

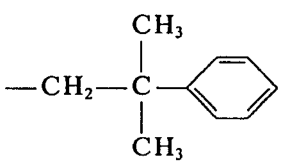

X Sauerstoff oder Schwefel und

$Y_1$ und $Y_2$ je Wasserstoff oder $-Sn(R)_3$ bedeuten, dadurch gekennzeichnet, daß man eine Verbindung der Formel

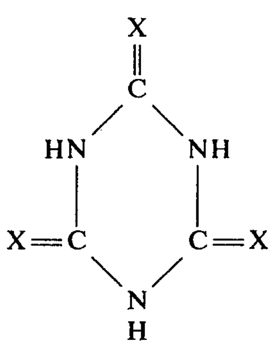

mit einer Verbindung der Formel

$HOSn(R)_3$ bzw. $(R)_3Sn-O-Sn(R)_3$

umsetzt, worin R und X die oben angegebene Bedeutung haben.

**Claims for the Contracting States: DE, GB, FR, CH, LI, IT, NL, BE**

1. A triorganotin isocyanuric compound of the formula

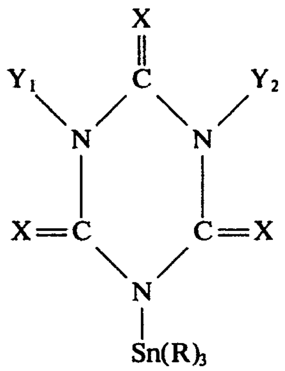

(I)

wherein
R is cyclohexyl or

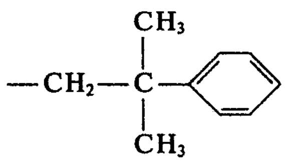

X is oxygen or sulfur, and
$Y_1$ and $Y_2$ are each hydrogen or $-$ $Sn(R)_3$.

2. A compound according to claim 1, wherein
R is cyclohexyl or

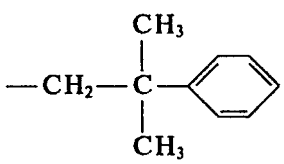

X is oxygen or sulfur, and
$Y_1$ and $Y_2$ are each $-Sn(R)_3$.

3. The compound according to claim 2 of the formula

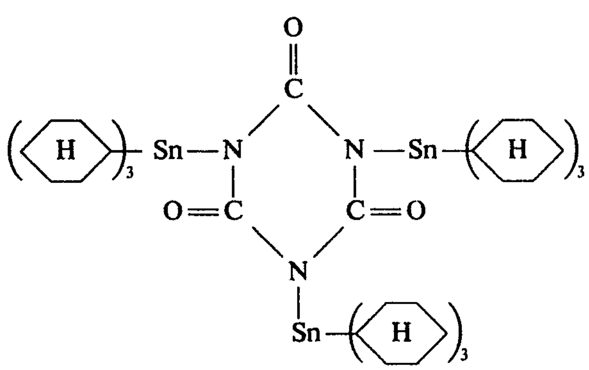

4. The compound according to claim 2 of the formula

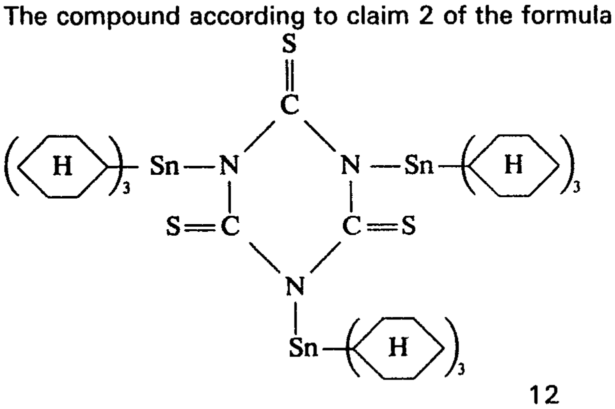

5. The compound according to claim 2 of the formula

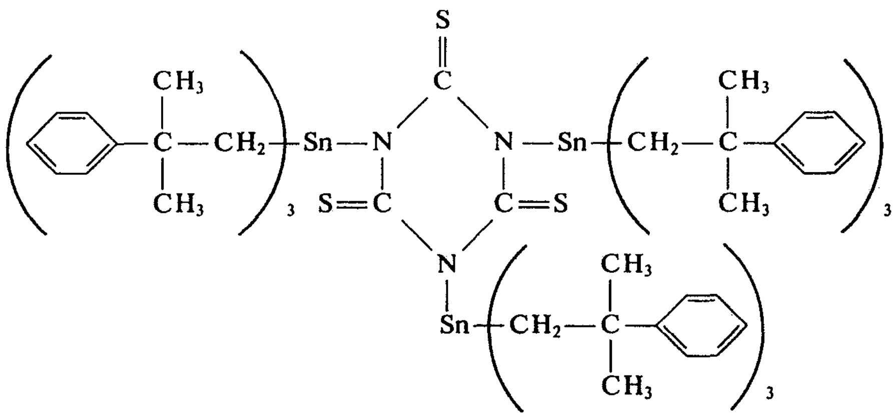

6. A process for the preparation of a compound according to claim 1, which process comprises reacting a compound of the formula

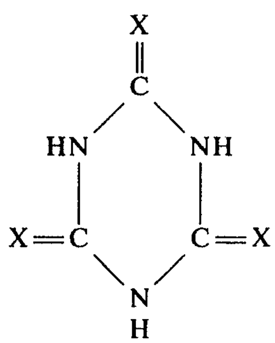

with a compound of the formula

$HOSn(R)_3$ or $(R)_3Sn-O-Sn(R)_3$

wherein R and X are as defined in claim 1.

7. A pesticidal composition which contains as active ingredient a compound according to claim 1.

8. A method of controlling various pests on animals and plants, and also of regulating plant growth, which method comprises applying thereto or to the locus thereof an effective amount of a compound according to claim 1.

9. A method according to claim 8 of controlling insects and members of the order Acarina.

**Claims for the Contracting State: AT**

1. A pesticidal composition which contains as active ingredient a triorganotin isocyanuric compound of the formula

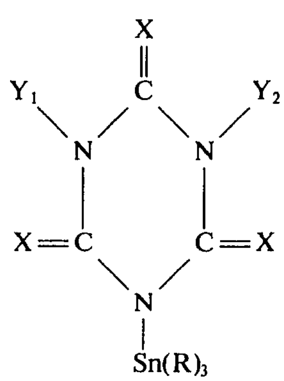

(I)

wherein
R is cyclohexyl or

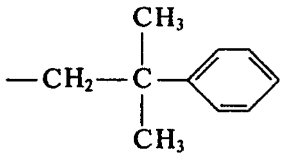

X is oxygen or sulfur, and
$Y_1$ and $Y_2$ are each hydrogen or $-Sn(R)_3$.

2. A composition according to claim 1, wherein in the formula of the active ingredient
R is cyclohexyl or

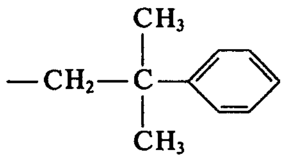

X is oxygen or sulfur, and
$Y_1$ and $Y_2$ are each $-Sn(R)_3$.

3. A composition according to claim 2, which contains as active ingredient the compound of the formula

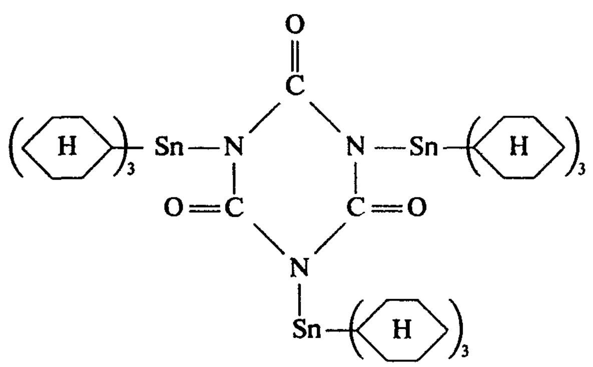

4. A composition according to claim 2, which contains as active ingredient the compound of the formula

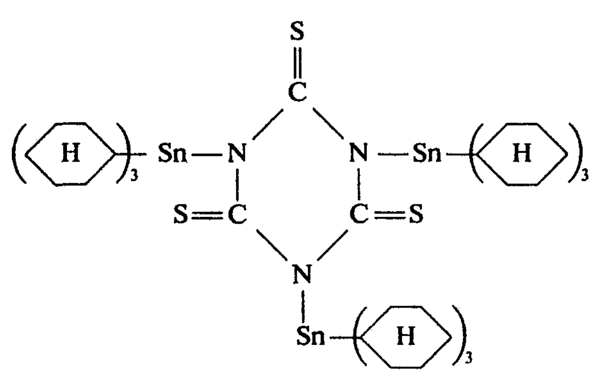

5. A composition according to claim 2, which contains as aktive ingredient the compound of the formula

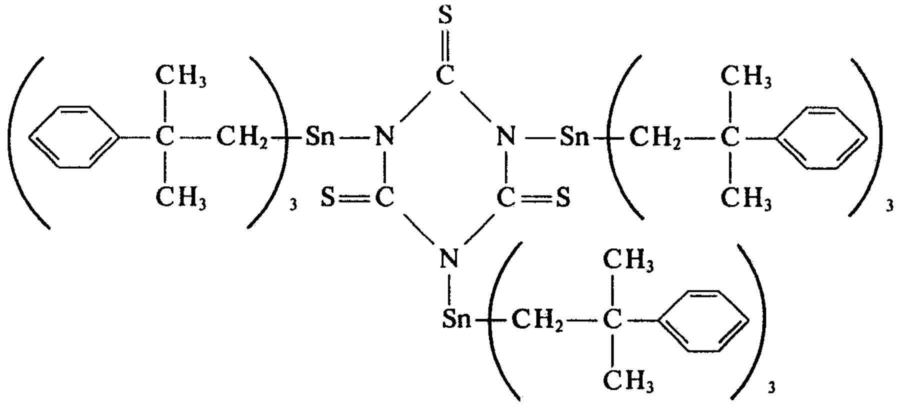

6. A method of controlling various pests on animals and plants, and also of regulating plant growth, which method comprises applying thereto or to the locus thereof an effective amount of a compound according to claim 1.

7. A method according to claim 6 of controlling insects and members of the order Acarina.

8. A process for the preparation of a triorganotin isocyanuric compound of the formula

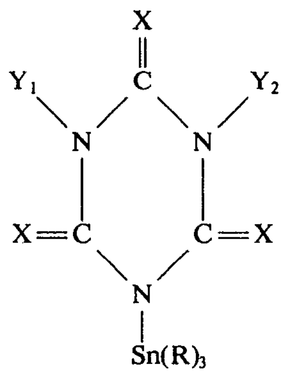

(I)

wherein

R is cyclohexyl or

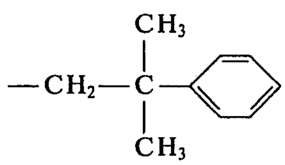

X is oxygen or sulfur, and

$Y_1$ and $Y_2$ are each hydrogen or $Sn(R)_3$, which process comprises reacting a compound of the formula

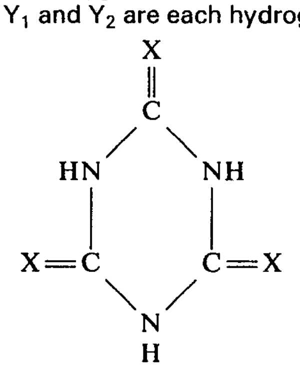

with a compound of the formula

$HOSn(R)_3$ or $(R)_3Sn-O-Sn(R)_3$

wherein R and X are as defined above.

**Revendications pour les Etats Contractants: BE, CH, LI, DE, FR, GB, IT, NL**

1. Composés triorganoétain d'isocyanure de formule

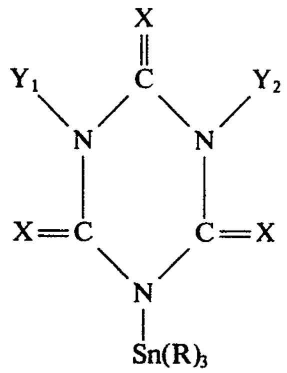

(I)

où R représente un cyclohexyle ou

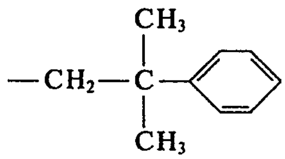

X représente un oxygène ou un soufre et
$Y_1$ et $Y_2$ représentent chacun un hydrogène ou $-Sn(R)_3$.

2. Composé selon la revendication 1, où
R représente un cyclohexyle ou

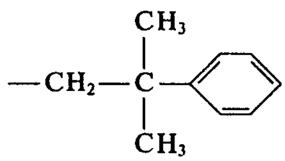

X représente un oxygène ou un soufre et
$Y_1$ et $Y_2$ représentent chacun $-Sn(R)_3$.

3. Le composé selon la revendication 2 de formule

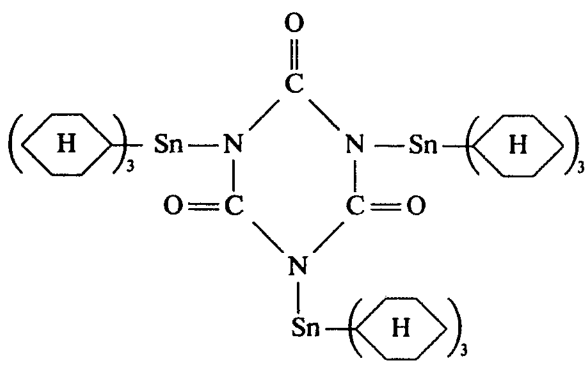

4. Le composé selon la revendication 2 de formule

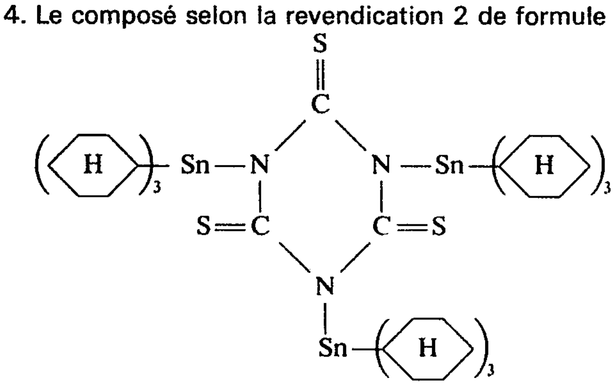

5. Le composé selon la revendication 2 de formule

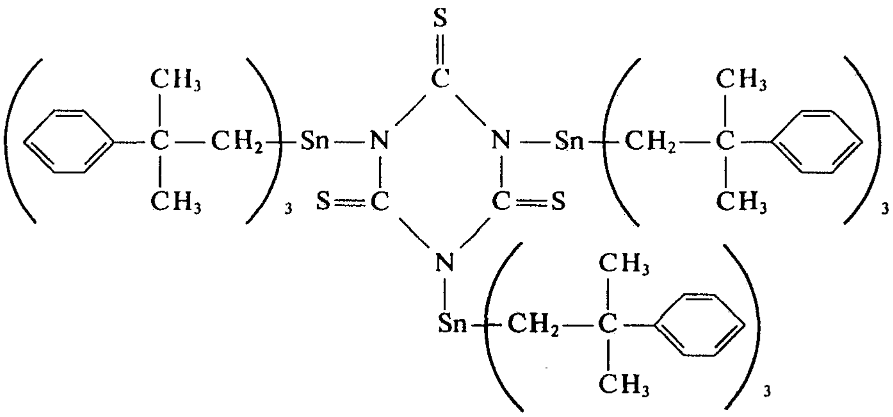

6. Procédé de préparation de composés selon la revendication 1, caractérisé en ce qu'on fait réagir un composé de formule

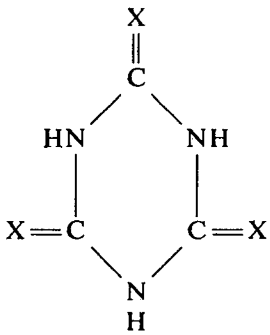

avec un composé de formule

$HOSn(R)_3$ ou $(R)_3Sn-O-Sn(R)_3$

où R et X ont la signification donnée dans la revendication 1.

7. Agent de lutte antiparasitaire contenant comme composant actif un composé selon la revendication 1.

8. Application d'un composé selon la revendication 1 pour lutter contre différentes espèces de parasites chez les animaux et les plantes ainsi que pour régler la croissance des plantes.

9. Application selon la revendication 8 pour combattre les insectes et les représentants de l'ordre des acariens.

**Revendications pour l'Etat Contractant: AT**

1. Agent de lutte antiparasitaire contenant comme composant actif un composé triorganoétain d'isocyanure de formule

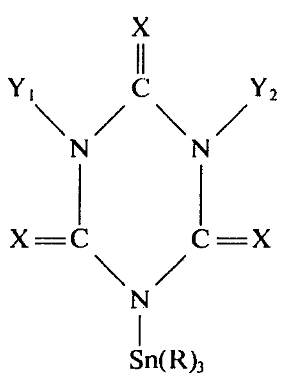

(I)

où R représente un cyclohexyle ou

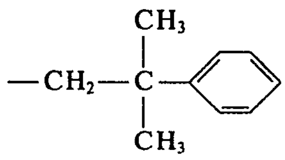

X représente un oxygène ou un soufre et $Y_1$ et $Y_2$ représentent chacun un hydrogène ou $-Sn(R)_3$.

2. Agent selon la revendication 1, où dans la formule du composé actif R représente un cyclohexyle ou

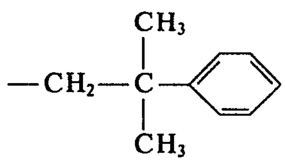

X représente un oxygène ou un soufre et $Y_1$ et $Y_2$ représentent chacun $-Sn(R)_3$.

3. Agent selon la revendication 2 contenant comme composant actif le composé de formule

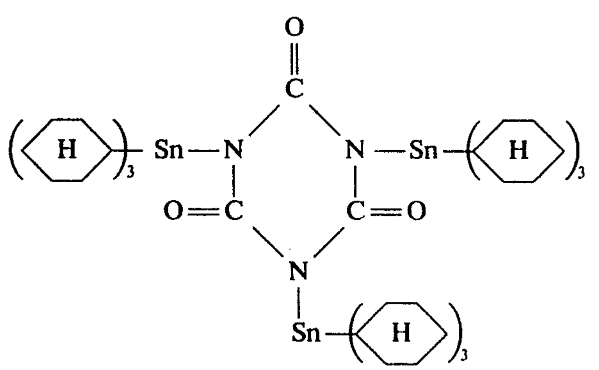

4. Agent selon la revendication 2 contenant comme composant actif le composé de formule

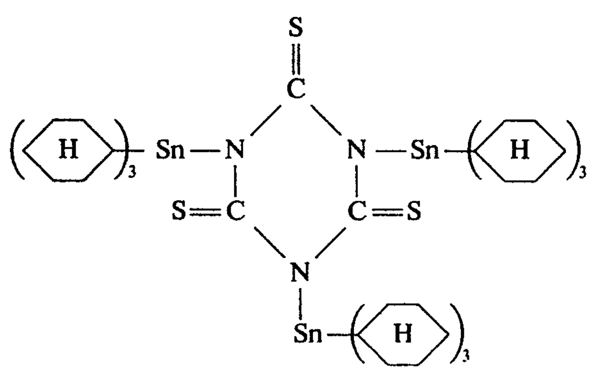

5. Agent selon la revendication 2 contenant comme composant actif le composé de formule

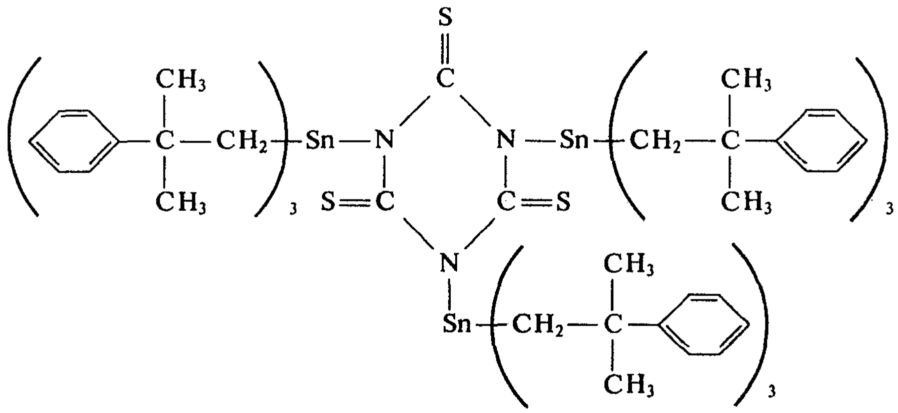

6. Application de l'agent selon la revendication 1 pour combattre différentes espèces de parasites chez les animaux et les plantes ainsi que pour régler la croissance des plantes.

7. Application selon la revendication 6 pour combattre les insectes et les représentants de l'ordre des acariens.

8. Procédé de préparation de composés triorganoétain d'isocyanure de formule

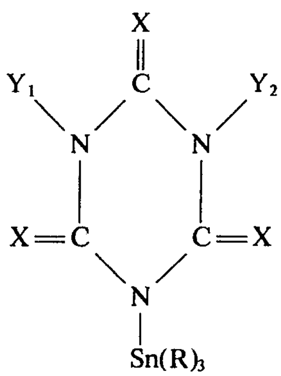

(I)

où R représente un cyclohexyle ou

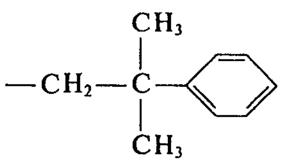

X représente un oxygène ou un soufre et

$Y_1$ et $Y_2$ représentent chacun un hydrogène ou $-Sn(R)_3$, caractérisé en ce qu'on fait réagir un composé de formule

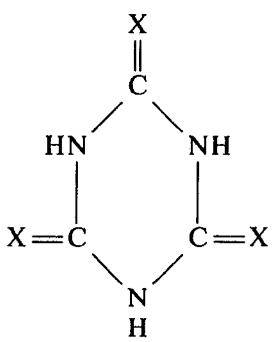

avec un composé de formule

$HOSn(R)_3$ ou $(R)_3Sn-O-Sn(R)_3$

où R et X ont la signification donnée ci-dessus.